# EUROPEAN PATENT APPLICATION

(11) **EP 1 287 817 A1**
(43) Date of publication of application: **05.03.2003**
(21) Application number: 01203305.6
(22) Date of filing: 31.08.2001
(51) Int. Cl.: A61K 9/00, A61K 45/00, A61K 45/06, A61P 15/00

(54) **Method of treating uterine leiomyomas and intravaginal drug delivery vehicle for use in such method**

(71) Applicant: Pantarhei Bioscience B.V., 3700 AL Zeist (NL)
(72) Inventor: Coelingh Bennink, Herman Jan Tijmen, 3971 BE Driebergen (NL); Thijssen, Joseph Helenus Hubert, 3981 TA Bunnik (NL)
(74) Representative: Jorritsma, Ruurd

(57) **Abstract**

The present invention relates to a method of treating uterine leiomyomas (fibroids or myomas) in mammal females. More particularly the present invention relates to such a method comprising intravaginal administration of a drug delivery vehicle to a female mammal suffering from uterine leiomyomas, said drug delivery vehicle containing an estrogen activity suppressant selected from the group consisting of aromatase inhibitors, anti-estrogens and mixtures thereof, wherein the method provides the estrogen activity suppressant in a therapeutically effective dosage to inhibit the growth and/or to achieve atrophy of the myomas. The intravaginal administration of estrogen activity suppressant, in comparison to other routes of administration, achieves the desired clinical effect at lower dosage levels and/or without seriously affecting overall estrogen blood serum levels.

Another aspect of the invention is concerned with a drug delivery vehicle for intravaginal use, which drug delivery vehicle comprises at least 10 µg of an aromatase inhibitor and/or at least 10 µg of an anti-estrogen and pharmaceutically acceptable excipient.

## Description

### TECHNICAL FIELD

The present invention relates to a method of treating uterine leiomyomas (fibroids or myomas) in mammal females. More particularly the present invention relates to such a method comprising intravaginal administration of a drug delivery vehicle to a female mammal suffering from uterine leiomyomas, said drug delivery vehicle containing an estrogen activity suppressant selected from the group consisting of aromatase inhibitors, anti-estrogens and mixtures thereof, wherein the method provides the estrogen activity suppressant in a therapeutically effective dosage to inhibit the growth and/or to achieve atrophy of the myomas.

Another aspect of the invention is concerned with a drug delivery vehicle for intravaginal use, which drug delivery vehicle comprises at least 10 µg of an aromatase inhibitor and/or at least 10 µg of an anti-estrogen and pharmaceutically acceptable excipient.

### BACKGROUND OF THE INVENTION

Uterine leiomyomas (fibroids or myomas), benign clonal tumours, arise from smooth-muscle cells of the human uterus. They are clinically apparent in up to 25% of women and are the single most common indication for hysterectomy. They cause significant morbidity, including prolonged and heavy menstrual bleeding, pelvic pressure and pain, urinary problems, and, in rare cases, reproductive dysfunction.

The pathophysiology of myomas is not well understood. However, both genetic predisposition and steroid hormone concentrations have a role in the development and growth of these benign tumours. In addition, growth factors play an important role in fibrotic processes and angiogenesis. At least two distinct steps to myoma formation can be identified. First, normal myocytes have to be transformed into abnormal myocytes and secondly, these abnormal cells subsequently have to grow into clinically apparent tumours. Microscopic myomas have a high prevalence, meaning that the first process is quite common. The subsequent growth occurs via clonal expansion. A variety of chromosomal subgroups have been found, implying that myomas can be seen as a common phenotype resulting from several different genetic events.

Myomas are found submucosally (beneath the endometrium), intramurally (within the myometrium) and subserosally (projecting out of the serosal compartment of the uterus), but mostly are mixed forms of these 3 different types.

The presence of estrogen receptors in leiomyoma cells has been studied by Tamaya et al. (Acta Obstet Gynecol Scand 1985; 64(4);307-9). They have shown that the ratios of estrogen receptor compared to progesterone and androgen receptor levels were higher in leiomyomas than in the corresponding normal myometrium.

Surgery has long been the main treatment for myomas. Women who have completed childbearing often undergo a hysterectomy, because this eliminates both the symptoms and the chance of recurrence. For women who desire future pregnancies or for other reasons wish to retain the uterus, other options are available. Myomectomy (removal of the myomas with uterine conservation) is widely done. However, the disadvantage of this treatment is the risk of new myoma formation.

Medical therapies that have been proposed to treat myomas include administration of a variety of steroids such as the androgenic steroids Danazol or Gestrinone, GnRH agonists and progestogens. Both Danazol and Gestrinone induce amenorrhea, thus controlling myoma-related menorrhagia. In addition, Gestrinone causes uterine volume reduction. Both these drugs have pronounced side effects, like decreasing the bone mineral density, weight-gain, hirsutism and acne. The GnRH agonists produce a significant reduction in uterine size, however, these drugs also cause bone loss and other symptoms of hypoestrogenism. In addition, once the GnRH agonists are discontinued, the size of the uterus increases to pretreatment volume. Therefore, the GnRH agonists are primarily used to temporise or to prepare women for surgery. The use of progestogens for the treatment of myomas is ineffective for many women. In addition, progestogens also induce hypoestrogenism.

Because of the induction of a hypoestrogenic milieu, the aforementioned medical therapies cannot be administered for more than 6 months, in particular not because of the risk of pronounced osteoporosis.

Several studies looked at long term medical therapy of myomas, based on a regimen wherein first downregulation to a hypogonatropic hypogonadal state is achieved through administration of a GnRH agonist and subsequently an estrogen and a progestogen are administered to achieve a maintainable reduction in uterine volume and amenorrhea. A drawback of this long term therapy is that it effectively makes it impossible for females who undergo such treatment to become pregnant during the period of treatment.

US 4,888,331 relates to a method for the induction of labor, termination of pregnancy, as well as for the treatment of gynecological disorders, which method comprises administering an agent comprising a compound having anti-progestational activity and a compound having anti-estrogenic activity. The gynecological disorders endometriosis and dysmenorrhea are mentioned. It is also observed in US 4,888,331 that the compounds having anti-progestational and anti-estrogenic activities can be applied locally or topically, or administered enterally or parenterally and that suitable for local or topical application are, for example, vaginal suppositories or transdermal systems, such as skin plasters.

US 4,639,440 (Finchimica S.R.L.) is concerned with new therapeutic uses of cyproterone acetate. The latter compound was known to have anti-androgen activity, but it is said to be also capable of inhibiting the estrogen biosynthesis by acting on the aromatasic system involved in the transformation of testosterone into estrogens. The new therapeutic uses described in US 4,639,440 include the treatment of uterine fibromatosis and myomatosis. US 4,639,440 only mentions the oral administration of cyproterone acetate. The examples illustrate that only in exceptional cases the oral administration of cyproterone acetate will lead to the complete disappearance of the myomas.

The most important shortcoming of all existing medical therapies for the treatment of leiomyomas is that none of them is curative, i.e. they do not lead to disappearance of the myomas. In addition, they all suffer from side-effects that become more pronounced as the duration of the therapy increases.

Consequently there is a need for a long term medical therapy of uterine leiomyomas, particularly for pre- and peri-menopausal females, that does not suffer from serious side-effects. In addition there is a strongly felt need for a curative medical therapy that will obviate the need for surgical treatments such as hysterectomy and myomectomy.

### SUMMARY OF THE INVENTION

Surprisingly it was found that the aforementioned goals may be realised by a method of treatment that comprises intravaginal administration of a therapeutically effective dosage of an aromatase inhibitor and/or an anti-estrogen. Although we do not wish to be bound by theory, it is believed that myoma proliferation is the result of estrogen induced autostimulation, i.e. that myomas are capable of synthesising estrogens and that these estrogens will bind with the estrogen receptors within the same myoma, thereby triggering (further) proliferation of the myoma. By administering the present estrogen activity suppressant in a non-systemic way, it was found to be possible to stop this cascade of myoma proliferation without serious side-effects on the body's hormonal balance. Intravaginal administration was found to be particularly effective in achieving the goal of inhibiting myoma proliferation with a minimum of undesirable side-effects.

Aromatase is one of the P-450 enzymes. It catalyses the aromatisation of the A ring of the steroid skeleton in the steroid biosynthetic pathway starting from the cleavage of the side chain of cholesterol. To be more precise: aromatase catalyses the conversion of androstenedione to estrone as well as the conversion of testosterone to estradiol. Hence aromatase is a rate limiting enzyme for the biosynthesis of the latter estrogens. Sumitani et al. (Endocrinology 2000 Oct ; 141(10); 3852-61) characterized the expression of aromatase in leiomyomas and showed that leiomyomas overexpress aromatase P450 compared to normal myometrium.

Aromatase inhibitors are substances capable of inhibiting the catalytic activity of aromatase. In the context of the present invention aromatase inhibitors are substances that may be administered to animals, and especially humans, in non-toxic dosages so as to inhibit estrogen biosynthesis. At present a range of aromatase inhibitors is available and includes substances such as aminoglutethimide, anastrozole, exemestane, vorozole, letrozole, fadrozole, rogletimide, atamestane, formestane, liarozole, YM 511, TZA-2237, CGS 16949A and MEN 11066. Aromatase inhibitors primarily find application in methods of treating breast cancer. It has also been suggested that aromatase inhibitors may be used in the treatment of endometriosis.Anti-estrogens are substances which exhibit affinity for the mammalian estrogen receptors without triggering all of the responses that are characteristic of the interaction between estrogens and the same receptors. Thus, when administered in sufficiently high dosage, anti-estrogens will bind in appreciable amounts to estrogen receptors, thereby reducing the estrogen-receptor interaction. Consequently anti-estrogens can suitably be used to reduce or inhibit the impact of estrogens, hence the term "anti-estrogens". The term anti-estrogen as used throughout this document encompasses both anti-estrogens that trigger no estrogen receptor response at all as well as anti-estrogens that are capable of triggering a selective estrogen receptor response. An example of an anti-estrogen capable of triggering a selective estrogen receptor response are so called selective estrogen receptor modulators (SERM's). Suitable anti-estrogens for use in accordance with this invention include all conventional anti-estrogens, both steroidal and non-steroidal. Examples of suitable non-steroidal anti-estrogens include: tamoxifene, raloxifene, toremifene, idoxifene, droloxifene, nafoxidine, trioxifene, MER 25, ZM 189154, EM-652, EM-800, clomiphene, cyclophenil, lasofoxifene, arzoxifene, levormeloxifene, zindoxifene, LY 117018, LY 326315, ZK 119010, LY 357489, GW 5638, GW7604, TSE-424, FC1271a
Examples of suitable steroidal anti-estrogens include: ICI 164384, ICI 182780, RU 58668.

Walker et al (Preclinical evidence for therapeutic efficacy of selective estrogen receptor modulators for uterine leiomyoma; J Soc Gynecol Investig 2000; 7; 240-56) conducted preclinical studies in the Eker rat model to investigate the efficacy of SERM's as therapeutic agent for fibroids. Treatment with tamoxifen or LY 326315 by a subdermal pellet reduced the incidence of fibroids by 40-60% and reduced the size of the remaining fibroids.

It was found that intravaginal administration of an aromatase inhibitor and/or an anti-estrogen, in comparison to other routes of administration, achieves the desired clinical effect at lower dosage levels and/or without seriously affecting overall estrogen blood serum levels. Hence the present method may suitably be applied over a prolonged period of time, thereby significantly increasing the chance of achieving complete disappearance of the uterine leiomyomas. Furthermore intravaginal administration of the estrogen activity suppressant in accordance with the present invention offers the advantage that it has much less impact on the liver than, for instance, oral administration.

### DETAILED DESCRIPTION OF THE INVENTION

One aspect of the present invention is concerned with a method of treating leiomyomas in mammal females using a drug delivery vehicle, said method comprising intravaginal administration of the drug delivery vehicle to the female mammal suffering from leiomyomas, said drug delivery vehicle containing an estrogen activity suppressant capable of inhibiting the autosynthesis of estrogen or the interaction with the estrogen receptor in myomas, said estrogen activity suppressant being selected from the group consisting of aromatase inhibitors, anti-estrogens and mixtures thereof, wherein the method provides the estrogen activity suppressant in a therapeutically effective dosage to inhibit growth and/or to achieve atrophy of the myomas. The present method also encompasses the prophylactic treatment of leiomyomas. The term "drug delivery vehicle" as used throughout this document encompasses any pharmaceutical dosage systems suitable for delivering a pharmaceutically active principle to a mammal in such a way that the active principle can exert a pharmaceutical effect on said mammal. Because the present drug delivery vehicle is used in a method of intravaginal administration it should be suitable for vaginal insertion. Examples of drug delivery vehicles suitable for intravaginal use include suppositories, tampons, vaginal rings, tablets, capsules, gels and creams. In a preferred embodiment, said drug delivery vehicle is solid or semi-solid so as to facilitate insertion into the vagina. A preferred example of a solid vehicle is a tablet, capsule, suppository or a vaginal ring. It is noted that the solid drug delivery vehicle does not have to be entirely solid as, for instance, such a vehicle may suitably comprise a solid capsule and a liquid contained within said capsule.

The present method may successfully be applied to female mammals. Preferably these mammals include humans, cattle and pets. Most preferably the female mammal is a human female.

The present method provides the aromatase inhibitor and/or anti-estrogen in a therapeutically effective dosage to inhibit growth of myomas. By inhibition of growth of myomas is meant that existing myomas do not increase in volume as a result of proliferation and that no new myomas are formed. In a particularly preferred embodiment of the present invention the aromatase inhibitor and/or anti-estrogen is provided in a therapeutically effective dosage to achieve atrophy of the myomas. Most preferably the method provides said aromatase inhibitor and/or anti-estrogen in a therapeutically effective dosage to achieve full elimination of the myomas.

In principle any known aromatase inhibitor which is suitable for pharmaceutical application can be used in the present method. Examples of aromatase inhibitors which may be used in accordance with the invention are aminoglutethimide, anastrozole, exemestane, vorozole, letrozole, fadrozole, rogletimide, atamestane, formestane, liarozole, YM 511, TZA-2237, CGS 16949A, MEN 11066, precursors of the aforementioned substances, and mixtures thereof. Preferably the aromatase inhibitor is selected from the group consisting of anastrozole, exemestane, vorozole, letrozole and formestane, precursors of these substances, and mixtures thereof.

Examples of 'true' anti-estrogens that may be used in the present method include ICI 164384, ICI 182780, ZM 189154, EM-800, RU 58668, precursors of these anti-estrogens, and mixtures thereof.

In addition, anti-estrogens that exert SERM-like activity as well as their precursors may suitably be used in the present method. Such SERM-like anti-estrogens can suitably be selected from the group consisting of tamoxifen, raloxifene, toremifene, idoxifene, droloxifene, nafoxidine, trioxifene, MER 25, EM-652, clomiphene, cyclophenil, lasofoxifene, arzoxifene, levormeloxifene, zindoxifene, LY 117018, LY 326315, ZK 119010, LY 357489, GW 5638, GW 7604, TSE-424, FC1271a and mixtures thereof. It has been reported (Osteoporosis Conference Scrip No. 1812/13 Apr. 16/20, 1993, p. 29) that raloxifene (6-hydroxy-2-(4-hydroxyphenyl)-3-[4-(2-piperidinoethoxy) benzoyl] benzo[b] thiophene) mimics the favourable action of estrogen on bone and lipids but, unlike estrogen, has minimal uterine stimulatory effect. (Breast Cancer Res. Treat. 10(1). 1987 p 31-36 Jordan, V. C. et al.).

Most preferably the anti-estrogen used in accordance with the invention is selected from the group consisting of ICI 164384, ICI 182789, raloxifene, tamoxifen, precursors of these substances, and mixtures thereof.

In another preferred embodiment of the invention the method of treating leiomyomas comprises co-administration of an estrogen in a therapeutically effective amount to reduce possible symptoms of hypoestrogenism resulting from the administration of the estrogen activity suppressant. As mentioned herein before, the present method offers the advantage that, due to the non-systemic and/or relatively low intravaginal dosage of the estrogen activity suppressant, it will produce significantly less symptoms of hypoestrogenism than known methods for treating leiomyomas. However, in particular during prolonged treatment, certain symptoms of hypoestrogenism, such as hot flushes, may become manifest. These symptoms may suitably be suppressed by co-administering estrogen in a therapeutically effective dosage to compensate for the estrogen deficiency symptoms occurrring at other parts of the body than the uterus, resulting from the intravaginal administration of the estrogen activity suppressant. We have unexpectedly found that, in particular if the estrogen is administered systemically (e.g. orally), the blood serum estradiol level may be restored to its usual level, without the administered estrogen having a significant effect on the myomas' proliferation. In a preferred embodiment of the present method estrogen is co-administered with the estrogen activity suppressant so as to maintain the estrogen blood serum level of the female at a level which is equivalent to at least 30 pg 17β-estradiol/ml. Most preferably the 17β-estradiol blood serum level of the female is maintained at a level of at least 30 pg/ml.

The estrogen used in the present method is preferably selected from the group consisting of ethinyl estradiol, mestranol, quinestranol, estradiol, estrone, estran, estriol, estetrol, conjugated equine estrogens, precursors capable of liberating such an estrogen when used in the present method and mixtures thereof. Most preferably the estrogen is ethinyl estradiol, estradiol, or estetrol. In a preferred embodiment of the method of the invention the estrogen is selected from the group consisting of ethinyl estradiol, estradiol, estetrol, precursors of these estrogens and mixtures thereof, in an amount equivalent to 1-40 µg ethinyl estradiol (e.g. 0,5-5 mg 17β-estradiol).

In another preferred embodiment the present method comprises co-administration of an anti-progestogen in an effective amount to boost the atrophic effect of the anti-estrogen on the leiomyomas. Although applicants do not wish to be bound by theory it is believed that leiomyomas next to estrogen receptors also express progestogen receptors. These progestogen receptors, similar to the estrogen receptors, are deemed to play a crucial role in myoma proliferation. Thus the combined use of an anti-estrogen and an anti-progestogen is very effective in suppressing proliferation of myomas, but more importantly also in achieving atrophy of the uterine leiomyomas. In a particularly preferred embodiment also the anti-progestogen is administered intravaginally. Also in case of anti-progestogen the intravaginal administration offers the advantage that, in comparison to systemic administration, the same clinical effects, with less pronounced side-effects, are achieved at lower dosages.

In accordance with the present invention, the anti-progestogen is suitably administered in a daily amount of at least 10 µg. More preferably the minimum daily dosage is equivalent to an intravaginal daily dosage of at least 200 µg, most preferably at least 500 µg mifepristone. The maximum daily dosage of anti-progestogen is preferably equivalent to an intravaginal daily dosage of less than 500 mg mifepristone, more preferably of less than 50 mg mifepristone and most preferably of less than 25 mg mifepristone.

The anti-progestogen used in the present method can be a progesterone receptor antagonist or a pharmaceutically suitable agent that counteracts the normal biological activity of progesterone. Examples of anti-progestogens which can be employed in this invention are RU 486 (mifepristone, Roussel Uclaf, Paris; U.S. Pat. No. 4,386,085); Org 31710 [(6.alpha.,11.beta.,17.beta.)-11-(4-NMe.sub.2-phenyl)-6-Me-4',5'-dihydrospiro[oestra-4,9-diene-17,2(3'H)-furan]-3-one]; Org 33628[(11.beta.,17.alpha.)-(4-acetylphenyl)- 17,23-epoxy-19,24-dinorchola-4,9,20-trien-3-one]; onapristone (Schering Ag, Berlin; U.S. Pat. No. 4,780,461) and the steroids described in the following patents and patent applications: U.S. Pat. No. 4,609,651, especially the compound lilopristone (11β-(4-dimethylaminophenyl)-17β-hydroxy-17α-(3-hydroxy-prop-1-(Z)-enzyl-4,9(10) estradien-3-one); U.S. application Ser. No. 06/827,050, especially the compounds 11β-(4-acetylphenyl)-17β-hydroxy-17α-(1-propinyl)-4,9-estradien-3-one and 11β-(4-acetylphenyl)-17β-hydroxy-17α-(3-hydroxy-1(2)-propenyl)-4,9-estradien-3-one; U.S. application Ser. No. 07/283,632; published European patent application EP-A 04042831; published PCT application WO 91/14704; and other anti-progestogens, e.g., U.S. Pat. No. 4,891,368. Most preferably the anti-progestogen used in the present method is mifepristone and/or a precursor thereof.

Throughout this document by precursors of an active ingredient are meant components capable of liberating the active ingredient when used in the present method, particularly after intravaginal and/or, in the case of estrogen, following oral administration, e.g. as a result of metabolic conversion of the precursor substance.

It is to be understood that the present invention not only encompasses the use of the active principles specifically mentioned in this application, but also the use of metabolites of these components that display comparable functionality. In this context it is noted that, for instance, estriol is a metabolite of 17beta-estradiol. Both anti-estrogens and aromatase inhibitors may suitably be used as estrogen activity suppressants in the present method. It is preferred, however, for the estrogen activity suppressant to be an aromatase inhibitor. In particular when the estrogen activity suppressant is used in combination with an estrogen, the use of an aromatase inhibitor offers the advantage that it allows the interaction of said components with estrogen receptors in non-uterine tissue. Thus symptoms of hypoestrogenism are relatively easy to suppress when an aromatase inhibitor is used.

The drug delivery vehicle used in the present method is preferably administered intravaginally at intervals of between 12 hours and 90 days. More preferably said vehicle is administered between once a day or once a month. It can be advantageous to use a slow release drug delivery vehicle, particularly a vehicle that, following intravaginal administration, is capable of releasing the preferred daily dosage amounts during a period of at least 5, or more preferably at least 10 days, without intermediate replenishment.

In another preferred embodiment the present method comprises intravaginal administration of the estrogen activity suppressant in an amount which in the case the suppressant is an aromatase inhibitor is equivalent to a daily dosage of less than 25 mg vorozole, preferably of more than 10 µg vorozole and more preferably in an amount equivalent to a daily dosage of 25 µg to 2,5 mg vorozole, and in the case the suppressant is an anti-estrogen is equivalent to a daily dosage of less than 600 mg raloxifene, preferably of more than 250 µg raloxifene and more preferably in an amount equivalent to a daily dosage of 600 µg to 60 mg raloxifene. In a particularly preferred embodiment of the invention the aromatase inhibitor is administered in an amount which is equivalent to less than 1.25 mg, even more preferably less than 1.0 mg vorozole. Similarly, it is particularly preferred to administer the anti-estrogen in an amount which is equivalent to less than 30 mg, more preferably even less than 20 mg raloxifene. The phrase "equivalent to a daily dosage" should not be interpreted restrictedly. For instance, the above mentioned requirement that the administration of the present drug delivery vehicle is to provide the equivalent of a daily dosage of 25 µg to 2,5 mg vorozole, encompasses a protocol wherein vorozole is administered once a week, provided the weekly dosage is between 175 µg and 17,5 mg, i.e. such that the average daily dose is between 25 µg and 2,5 mg.

An important advantage of the present method of treating leiomyomas is that, unlike existing methods, it may be employed for prolonged periods of time, without any serious side-effects. Hence in a preferred embodiment the present method comprises uninterrupted intravaginal administration of the drug delivery vehicle for a period of at least 3 months, preferably at least 6 months. It is noted that with the present method usually a treatment period of at least 3-6 months is necessary to obtain significant atrophy or complete disappearance of myomas. Leiomyomas is much more frequently observed in pre-menopausal than in postmenopausal women. Hence the benefits of the present method are particularly appreciated when said method is applied to pre- and peri-menopausal females.

Another aspect of the invention is concerned with a drug delivery vehicle for intravaginal use, comprising at least 10 µg of an aromatase inhibitor and/or at least 10 µg of an anti-estrogen and pharmaceutically acceptable excipient. Preferably the amount of aromatase inhibitor exceeds 20 µg and/or the amount of anti-estrogen exceeds 50 µg. Usually the amount of aromatase inhibitor and/or the amount of anti-estogen will not exceed 600 mg, preferably they will not exceed 100 mg. Most preferably the drug delivery device contains aromatase inhibitor in an amount equivalent to at least 25 µg vorozole and/or anti-estrogen in an amount equivalent to at least 600 µg raloxifene. The drug delivery vehicle according to the invention may be a tablet, capsule, gel, cream, film, suppository, tampon or a vaginal ring.

In a preferred embodiment the drug delivery vehicle additionally contains an estrogen in an amount equivalent to an oral dosage of at least 1 µg ethinyl estradiol. In yet another preferred embodiment the drug delivery vehicle additionally contains at least 10 µg of an anti-progestogen. Preferably the delivery vehicle contains anti-progestogen in an amount equivalent to at least 200 µg, more preferably at least 500 µg mifepristone. Usually the amount of anti-progestogen will not exceed 500 mg, preferably it will not exceed 100 mg. The vaginal suppository may suitably be based on a hard fat, preferably a hard fat having a hydroxy value not exceeding 50. The term 'hard fat' means a mixture of glycerides whose constituent fatty acids are straight-chain saturated fatty acids of 8 to 18 carbon atoms, and examples of such hard fat are listed on Martindale The Extra Pharmacopeia, 28ed., p. 1067, London, The Pharmaceutical Press, 1982). The vaginal suppositories according to the present invention may be manufactured by melting the hard fat adding the pharmaceutically active principles, mixing them thoroughly, pouring the composition into suppository moulds in predetermined uniform quantities and cooling them.

In another embodiment, the invention provides a tampon device for delivering the estrogen activity suppressant to the vaginal epithelium, said tampon comprising an absorbent material which has been soaked with a pharmaceutically acceptable solution of the estrogen activity suppressant. A retrieval string or tape may be connected to the tampon device so as to facilitate easy removal. When administered the tampon contacts the vaginal epithelium for delivery of the agent.

In yet another embodiment the delivery vehicle may be a vaginal ring. Vaginal rings are torous shaped devices designed to deliver a relatively constant dose of drug to the vagina usually over a period of weeks to months. Typically, they are made of a poly EVA elastomer and contain a drug released by diffusion though the elastomer. The most common commercial applications have been to deliver low doses of steroids for post-menopausal vaginal conditions. They have also been under development for use in contraception and hormone replacement therapy. Vaginal rings have also been used to administer spermicides, as well as a variety of locally or systematically active medicaments.

The use of a vaginal ring to deliver drugs requires a ring design that regulates the release rate so as to provide the user with the appropriate daily dose. Among the important factors governing release are the solubility of the drug in the ring elastomer, the surface area of the drug reservoir, the distance the drug must diffuse through the ring body to reach its surface and the molecular weight of the drug.

If very high release rates are desired, they can be attained by a drug load at the ring surface as is characteristic of the homogeneous matrix ring design. This design, however, suffers from rapidly declining release rates as the distance the drug must travel to reach the ring surface increases as the drug load near the surface is depleted. If moderately high release rates are needed to provide the appropriate dose, a design which modulates release rate by imposing a layer of drug-free elastomer between the drug reservoir and the ring exterior is appropriate. This may be attained by coating a homogeneous ring, or to conserve drug, by incorporating a drug-free core, a shell design may be used. If an even lower release rate is desired, the drug may be confined to a small diameter at the center of the ring ("core ring"). Numerous types of vaginal rings have been described in the patent and non-patent literature alike.

Yet another aspect of the present invention relates to a pharmaceutical kit comprising (i) at least one drug delivery vehicle for intravaginal use, comprising at least 10 µg of an aromatase inhibitor and/or at least 10 µg of an anti-estrogen and pharmaceutically acceptable excipient and (ii) a medicament for oral administration containing estrogen in an amount equivalent to at least 1 µg ethinyl estradiol and pharmaceutically acceptable excipient. As explained herein before the combination of intravaginal administration of estrogen activity suppressant and oral administration of estrogen was found to be particularly effective in treating leiomyomas with minimum side-effects. Preferably the estrogen containing medicament is for once daily oral administration.

The dosage units in the aforementioned kit may advantageously contain an anti-progestogen. Said anti-progestogen may suitably incorporated in either or both the intravaginal drug delivery device and the oral medicament. Preferably the anti-progestogen is incorporated in the intravaginal drug delivery device in an amount of at least 10 µg.

The invention is further illustrated by means of the following examples.

### EXAMPLES

### Example 1

A clinical efficacy study is conducted in 10 women with uterine fibroids, who have to undergo a myomectomy or a hysterectomy. Eight women are randomised to a treatment group receiving a daily dosage of 500 µg vorozole intravaginally. Two women are randomised to a placebo group. Before the start of the study the number and size of the fibroids is assessed by ultrasonography for each participant. This procedure is repeated every 2 weeks after the start of the study until the female undergoes myomectomy or hysterectomy. During the period of study participants are recording symptoms of hypoestrogenism in a diary. After 5-16 weeks of vaginally administered vorozole, the size of the fibroids is reduced in all participants who receive vorozole. Reductions in total fibroids volume of up to 75% are observed. A few women experience symptoms of hypoestrogenism. The size of the fibroids in the women in the placebo group is found to have increased during the period the women receive placebos.

### Example 2

Example 1 is repeated, using instead of 500 µg vorozole a daily intravaginal dosage of 10 mg raloxifene. A signifant reduction in size of the fibroids is observed in the females receiving raloxifene, whereas no such reduction is observed in the control group.

### Example 3

Example 1 is repeated without a control group and with the exception that participants (who were not using an oral contraceptive during the period of the study) additionally receive a daily oral dosage of 1 mg 17-β estradiol. The participants receiving the combination of vaginal vorozole and oral estrogen show significant fibroid atrophy during the treatment and record less symptoms of hypoestrogenism than the females described in example 1, who only receive vorozole.

### Example 4

Example 1 is repeated without a control group and with the exception that the participants who receive vorozole additionally receive a daily intravaginal dosage of 10 mg mifepristone. The participants receiving the combination of vorozole and mifepristone show a larger reduction of fibroid size than the participant of example 1 who received only vorozole.

## Claims

1. Drug delivery vehicle for use in a method of treating leiomyomas in mammal females, said method comprising intravaginal administration of the drug delivery vehicle to the female mammal suffering from leiomyomas, said drug delivery vehicle containing an estrogen activity suppressant selected from the group consisting of aromatase inhibitors, anti-estrogens and mixtures thereof, wherein the method provides the estrogen activity suppressant in a therapeutically effective dosage to inhibit growth and/or to achieve atrophy of the myomas.

2. Drug delivery vehicle according to claim 1, wherein the method comprises co-administration of estrogen in a therapeutically effective amount to reduce symptoms of hypoestrogenism resulting from the administration of the estrogen activity suppressant.

3. Drug delivery vehicle according to claim 1 or 2, wherein the estrogen activity suppressant is an aromatase inhibitor.

4. Drug delivery vehicle according to any one of claims 1-3, wherein the method comprises co-administration of an anti-progestogen in an effective amount to boost the atrophic effect of the anti-estrogen on the leiomyomas.

5. Drug delivery device according to any one of claims 1-4, wherein during treatment the blood serum estrogen level of the female is maintained at a level which is equivalent to at least 30 pg 17 β-estradiol /ml.

6. Drug delivery vehicle according to any one of claims 1-5 wherein the method comprises uninterrupted intravaginal administration of the drug delivery vehicle for a period of at least 3 months, preferably at least 6 months.

7. Drug delivery vehicle according to claim 2, wherein the estrogen is co-administered orally.

8. Drug delivery vehicle for intravaginal use, comprising at least 10 µg of an aromatase inhibitor and/or at least 10 µg of an anti-estrogen and pharmaceutically acceptable excipient.

9. Drug delivery vehicle according to claim 8, wherein the drug delivery vehicle additionally comprises at least 10 µg of an anti-progestogen.

10. Drug delivery vehicle according to claim 8 or 9, wherein the drug delivery vehicle is a suppository, a tampon or a vaginal ring.

11. Pharmaceutical kit comprising a drug delivery vehicle according to any one of claims 8-10 and a plurality of oral dosage units comprising an estrogen in an amount equivalent to a daily oral dosage of at least 1 µg ethinyl estradiol.
